# EUROPÄISCHE PATENTSCHRIFT

(11) **EP 1 740 566 B1**
(45) Veröffentlichungstag und Bekanntmachung des Hinweises auf die Patenterteilung: **08.08.2007**
(21) Anmeldenummer: 05714937.9
(22) Anmeldetag: 02.02.2005
(51) Int. Cl.: C07D 309/10, A61K 31/65, C12P 29/00, A61P 31/04

(54) **HKI10311129, NEUES ANTIBIOTIKUM, VERFAHREN ZU DESSEN HERSTELLUNG UND DESSEN VERWENDUNG**
HKI10311129, NOVEL ANTIBIOTIC, METHOD FOR PRODUCING THE SAME AND THE USE THEREOF
NOUVEL ANTIBIOTIQUE HKI10311129, PROCEDE DE PRODUCTION ET UTILISATION DUDIT ANTIBIOTIQUE

(30) Priorität: 27.02.2004 DE 102004010219
(43) Veröffentlichungstag der Anmeldung: 10.01.2007
(73) Patentinhaber: Leibniz-Institut für Naturstoff-Forschung und Infektionsbiologie e.V. Hans-Knöll-Institut, 07745 Jena (DE)
(72) Erfinder: MÖLLMANN, Ute, 07749 Jena (DE); HEROLD, Kerstin, 07745 Jena (DE); ROTH, Martin, 07749 Jena (DE); GROTH, Ingrid, 07751 Jena-Wogau (DE); GOLLMICK, Friedrich, 07743 Jena (DE); GRÄFE, Udo DI, (DE)
(74) Vertreter: Bock, Gerhard
(86) Internationale Anmeldenummer: PCT/DE2005/000186
(87) Internationale Veröffentlichungsnummer: WO 2005/082878

(56) Entgegenhaltungen:
- DE-A1- 10 065 606
- HEROLD, KERSTIN ET AL: "Biosynthesis of cervimycin C, an aromatic polyketide antibiotic bearing an unusual dimethylmalonyl moiety" ORGANIC & BIOMOLECULAR CHEMISTRY , 2(17), 2411-2414 CODEN: OBCRAK; ISSN: 1477-0520, 2004, XP002336421
- PATENT ABSTRACTS OF JAPAN Bd. 1995, Nr. 03, 28. April 1995 (1995-04-28) & JP 06 339395 A (MITSUBISHI KASEI CORP), 13. Dezember 1994 (1994-12-13) in der Anmeldung erwähnt

## Beschreibung

Bekanntermaßen werden Erreger bakterieller Infektionskrankheiten unter dem Einfluß der Antibiotikatherapie unter Selektionsdruck resistent, u.a. auch beschleunigt durch zu häufige und falsche Anwendung von Antibiotika. Besondere Probleme bereiten zunehmend Infelctionen, die durch mehrfach-resistente grampositive Bakterien, wie Staphylococcus aureus oder durch Glycopeptid-resistente Enterokokken hervorgerufen werden, die, wie im Fall des Hospitalismus, bei immun-geschwächten Patienten zu gefährlichen Erkrankungen führen können. Die bei diesen Keimen auftretenden Multiresistenzen, erschweren die Behandlung aufgrund nicht mehr zur Verfügung stehender Therapeutika. Vancomycin (ein Glykopeptid) ist das Reserveantibiotikum gegen schwerwiegende Infektionen mit multiresistenten Staphylokokken (MRSA). Die vanA determinierte Vancomycinresistenz trat bisher nur bei Enterokokken (VRE) auf, ohne das extreme Multiresistenzproblem wie bei MRSA. Der lange Zeit gefürchtete interspezifische Transfer der vanA-Resistenz von Enterokokken auf Staphylokokken trat erstmals 2002 in den USA auf. Bei diesen Vancomycin-hochresistenten Staphylococcus aureus-Stämmen ist das vanA Gen in ein Multiresistenzplasmid integriert (Gottlieb, S. BMJ 326; 783(2003)).

Diese Entwicklungen und die teilweise unterschätzte Notwendigkeit der intensiven Suche nach neuen Strukturen und Targets führten zu einem Mangel an ausreichend wirksamen Therapeutika. Um die Heilung bakterieller Erkrankungen zu sichern und Infektionen mit Antibiotika-Resistenzen behandeln zu können, sind dringend neue antibiotisch wirksame Substanzen erforderlich (siehe z.B. Gräfe, Biochemie der Antibiotika, Spektrum Heidelberg 1992; S. Grabley, R. Thiericke & U. Gräfe, Drug Discovery from Nature, Springer, S. 281-301, 1999). Vor diesem Hintergrund gewinnen neue Antibiotika, welche die beschriebenen Resistenzbarrieren überwinden können, enorm an Bedeutung. Darüberhinaus sollten andere Nachteile, wie ungenügendes Wirkungsspektrum, ungünstige Pharmakokinetik und Nebenwirkungen der in Anwendung befindlichen Antibiotika überwunden werden.

Aufgabe der Erfindung ist es, einen neuen mikrobiellen Wirkstoff mit origineller Struktur und guten antimikrobiellen Eigenschaften, speziell gegen multiresistente Keime, zur Verfügung zu stellen.

Diese Aufgabe wird erfindungsgemäß dadurch gelöst, daß der Aktinomycetenstamm Streptomyces sp. HKI 0179 in einem flüssigen Nährmedium mit Kohlenstoff- und Stickstoffquelle sowie üblichen anorganischen Salzen fermentiert wird, bis sich der neue antimikrobielle Wirkstoff, nachfolgend HKI10311129 genannt, in der Kulturbrühe anhäuft und anschließend HKI10311129 in reiner Form daraus isoliert werden kann. Der Streptomycesstamm HKI 0179 wurde in der Deutschen Sammlung für Mikroorganismen und Zellkulturen (DSMZ) in Braunschweig, Mascheroder Wege 1, unter der Bezeichnung DSM 13059 hinterlegt.

HKI10311129 unterscheidet sich von den bisher bekannten Antibiotika durch seine chemische Struktur, deren Neuheit durch physilcochemische Messungen zweifelsfrei belegt wird (Tab. 1 und 2).

Von verwandten Strukturen, die in dem japanischen Patent 94 339 395, in CA 122, 237919g und in J. Antibiot., 45 (1992), 1974-6 beschrieben wurden, sind keine antimikrobiellen Wirkungen bekannt. Von einer weiteren verwandten Struktur, beschrieben in J. Antibiot. 51 (1998), 21-25, 26-32 ist zwar eine Aktivität gegen Methicillin resistente Staphylokokken bekannt, aber keine Aktivität gegen Enterokokken und besonders auch keine Aktivität gegen Stämme mit vanA determinierter Vancomycin-Resistenz.

Obwohl aus dem Streptomycesstamm DSM 13059 das Antibiotikum Altamiramycin schon bekannt ist (DE 100 65 606), wurde jetzt der neue antimikrobielle Wirkstoff HKI10311129 entdeckt, der überraschenderweise eine sehr starke antibakterielle Wirksamkeit gegen grampositive Bakterien besitzt, insbesondere gegen multiresistente Keime wie Staphylokokken und Enterokokken. HKI10311129 kann speziell als Heilmittel für Infektionen mit multiresistenten grampositiven Keimen, einschließlich solcher mit vanA determinierter Glykopeptidresistenz, bei Mensch und Tier eingesetzt werden.

Die Erfindung betrifft somit:
1. Eine Verbindung der Molmasse 1113, der Summenformel C₅₆H₇₅N0₂₂, der Strukturformel: 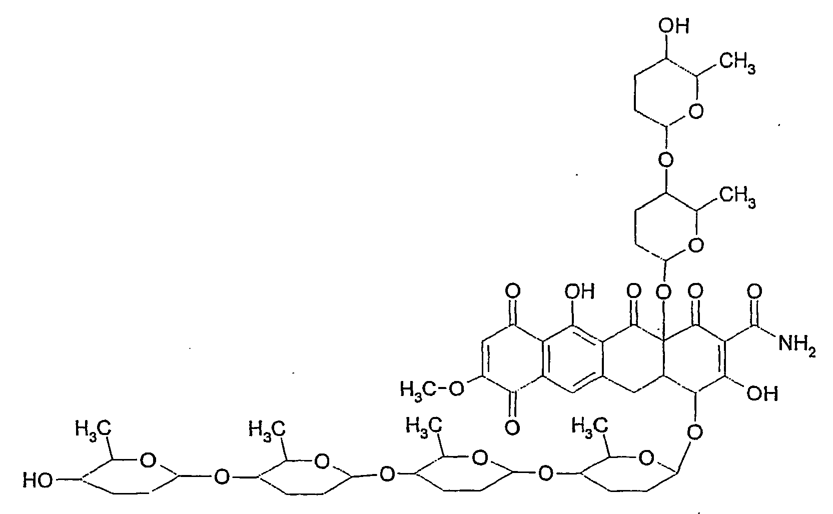 und den in Tabellen 1 und 2 aufgeführten physikochemischen Charakteristika, genannt HKI10311129.
2. Arzneimittel, enthaltend HKI10311129 nebst üblichen Träger- und Hilfsstoffen wie beispielsweise im "Remington's Pharmaceutical Sciences Handbook, Hack Pub. co., N. Y., USA", oder in Remington: The Science and Practice of Pharmacy by Alfonso R. Gennaro (Editor), A. L. Gennaro, oder im Handbook of Pharmaceutical Additives, Second Edition Pharmaceutical Additives Electronic Handbook-2002, Handbook of Pharmaceutical Additives, Second Edition Book and CD, Michael and Irene Ash, beschrieben.
   Die autiinfektiven Verbindungen können zur Verwendung als Lösung oder Suspension in pharmazeutisch akzeptablen Medien für eine topische oder parenterale Applikation, über intravenöse, subcutane oder intramuskuläre Injektionen, für intranasale Applikation; als Tablette, Kapsel oder Suppositorium zubereitet werden. Die Verbindungen können in Dosierungen von 0,1 -1000 mg/kg Körpergewicht eingesetzt werden.
3. Ein Verfahren zur Herstellung von HKI10311129, das dadurch gekennzeichnet ist, daß man Streptomyces sp. DSM 13059 in einem Nährmedium kultiviert, bis sich HKI10311129 in der Kulturbrühe anhäuft, und man es nachfolgend daraus isoliert.
4. Die Verwendung von HKI10311129 als pharmakologisch wirksamer Stoff, insbesondere als Antibiotikum, insbesondere gegen multiresistente und Vancomycin-resistente Bakterien.

Die Erfindung wird im folgenden detailliert beschrieben, insbesondere in ihren bevorzugten Ausführungsformen. Ferner wird sie durch den Inhalt der Patentansprüche bestimmt.

Definitionen:
- Die erfindungsgemäße Verbindung wird HKI10311129 genannt.
- Als Kulturbrühe wird das Nährmedium, welches das darin gewachsene Streptomyceten-Myzel enthält, bezeichnet.

Das erfindungsgemäße Antibiotikum HKI10311129 wird durch einen Streptomyces sp.-Stamm produziert, einem Isolat aus der "Grotta dei Cervi" in Süditalien. Dieser Stamm ist durch spezifische Merkmale charakterisiert (siehe unten).

Aufgrund von aufeinanderfolgenden Isolierungsschritten wurde von Streptomyces sp. DSM 13059 eine Kolonie isoliert, die das Antibiotikum HKI10311129 besonders effizient in der Kulturbrühe anhäuft. Ein Isolat von Streptomyces sp. DSM 13059 wurde bei der Deutschen Sammlung von Mikroorganismen und Zellkulturen GmbH in Braunschweig, Mascheroder

Weg 1B, 38124 Braunschweig, Deutschland, unter der Hinterlegungs-Nr. 13059 am 24.9.1999 gemäß den Bedingungen des Budapester Vertrages hinterlegt. Es kann wie folgt charakterisiert werden: LL-DAP im Peptidoglycan, Sporenträger vom Typ S, offene Spiralen, meist kurze 34, max. 6-7 Windungen und RA-Haken, Schlingen gerade, große Sporen, keine Melaninbildung, lösl. braunes Pigment auf allen ISP-Medien, dichtes, grau versportes LuftMyzel, braun - schwarzbraunes, auf ISP 4-Medium rötlich violettes SubstratMyzel, Wachstum und Sporulation auf allen ISP-Medien sehr gut.

In einem Medium mit einer Kohlenstoffquelle, einer Stickstoffquelle und üblichen Mineralsalzen produziert Streptomyces sp. DSM 13059 die erfindungsgemäße Verbindung HKI10311129. Anstelle des Stammes DSM 13059 können auch dessen Varianten und Mutanten eingesetzt werden. Als Varianten und Mutanten gelten alle jene Mikroorganismen der Spezies, die in der Lage sind, die erfindungsgemäßen Verbindungen zu synthetisieren. Solche Varianten und Mutanten können in an sich bekannter Weise durch physikalische Mittel, beispielsweise Bestrahlung mit Ultraviolett- oder Röntgenstrahlen oder chemische Mutagene, erzeugt werden.

Das Screening nach Mutanten und Varianten, die die erfindungsgemäße Verbindung synthetisieren, kann durch Bestimmung der biologischen Aktivität der in der Kulturbrühe angehäuften Wirkstoffe, beispielsweise durch Austesten der antibiotischen Wirkung auf bekannte Testkeime anhand der dem Fachmann bekannten Methoden und durch chromatographischen Nachweis durchgeführt werden.

Als bevorzugte Kohlenstoffquellen für die aerobe Fermentation eignen sich asssimilierbare Kohlenhydrate und Zuckeralkohole, wie z.B. Glucose, Lactose, Maltose, Glycerol, Mannitol oder deren Gemische sowie kohlenhydrathaltige Naturprodukte, wie Malzextrakt oder Melasse.

Als Stickstoffquellen eignen sich Aminosäuren, Peptide und Proteine sowie deren Abbauprodukte, wie Tryptone oder Peptone, ferner Fleischextrakte, gemahlene Samen, beispielsweise von Mais, Weizen, Bohnen, Sojabohnen oder der Baumwollpflanze, Destillationsrückstände der Alkoholherstellung, Fischmehle oder Hefeextrakte.

Die Kultivierung erfolgt aerob, beispielsweise submers unter Schütteln oder Rühren in Erlenmeyerkolben oder Fermentoren, gegebenenfalls unter Einleiten von Luft oder Sauerstoff.

Die Fermentation kann beispielsweise in Steilbrustflaschen, Erlenmeyer- oder Rundkolben verschiedener Volumina, in Glasfermentoren oder V₂A-Stahltanks durchgeführt werden.

Sie wird in einem Temperaturbereich zwischen 15 °C - 37 °C und einem pH-Wert zwischen 4 und 8 durchgeführt.

Der Mikroorganismus wird unter diesen Bedingungen 3 - 6 Tage kultiviert.

Die Fermentation kann im Labormaßstab (Kulturvolumina zwischen 100 ml und 200 ml), aber auch im Produktionsmaßstab (Volumina bis mehrere m³) durchgeführt werden.

Vorteilhafterweise kultiviert man in mehreren Stufen, d.h. zunächst werden eine oder mehrere Vorkulturen in einem flüssigen Nährmedium hergestellt, die dann in das eigentliche Produktionsmedium, die Hauptkultur, beispielsweise im Verhältnis 1:15 - 1:20, überimpft werden.

Die Vorkultur erhält man, indem man ein versportes Myzel von z.B. Hafermehl-Nährböden in eine Nährlösung überführt, beispielsweise durch Einimpfen von Myzel-bewachsenen Agarstücken, und diese 1 bis 2 Tage inkubiert.

Das versporte Myzel kann beispielsweise erhalten werden, indem man den Stamm etwa 7-10 Tage auf einem Nährboden, wie z.B. Hafermehl-Agar oder Sojamehl-Glucose-Agar, wachsen läßt.

Das Antibiotikum HKI10311129 ist in wechselnden Anteilen in der Kulturbrühe, sowie vorzugsweise im Myzel, enthalten.

Zum Testen der Wirkstoffkonzentration im Kulturmedium oder in den einzelnen Isolierungsstufen können die üblichen dem Fachmann vertrauten Methoden der biologischen Aktivitätsbestimmung, z.B. der Agardiffusions-Lochplattentest, angewendet werden.

Die Detektion bei der dünnschichtchromatografischen Auftrennung kann durch verschiedene Färbeagentien (beispielsweise Vanillin-Schwefelsäure) erfolgen.

Die Isolierung des Antibiotikums HKI10311129 aus der Kulturbrühe und dem Myzel erfolgt nach bekannten Methoden unter Berücksichtigung der chemischen, physikalischen und biologischen Eigenschaften der Produkte.

Zur Isolierung des Antibiotikums HKI10311129 aus submersen Kulturansätzen wird am Ende der Fermentation Kulturmedium und Myzel getrennt und das Myzel mit organischen Lösungsmitteln, wie Methanol oder Aceton, vorzugsweise Methanol, extrahiert.

Weitere Mengen an HKI10311129 werden durch Extraktion der vom Myzel abgetrennten Kulturlösung mit wasserunlöslichen organischen Lösemitteln, wie z.B. Essigsäureethylester, gewonnen.

Nach Einengen der Extrakte und gegebenenfalls Reextraktion des eingeengten Methanolextraktes des Myzels mit wasserunlöslichen organischen Lösungsmitteln, wie z.B. Essigsäureethylester oder Dichlormethan, erfolgt die Isolierung des Antibiotikums HKI10311129 unter Einsatz üblicher chromatographischer Adsorbentien bzw. Trägermaterialien, wie z.B. Kieselgel oder organophile Dextrangele.

Durch sequentielle Anwendung der Säulenchromatografie an Kieselgel, der Gelpermeations-Chromatografie an organophilen Dextrangelen unter Verwendung von polaren organischen Lösungsmitteln, wie z.B. Methanol, der Mitteldruckchromatografie an Kieselgel RP₁₈ unter Verwendung von Acetonitril/Wasser-Gemischen sowie der Hochleistungsflüssigkeitschromatografie unter Verwendung von Methanol/Wasser- und Acetonitril/Wasser-Gemischen wird schließlich reines HKI10311129 erhalten.

Die chemische Identität des Antibiotikums HKI10311129 wird durch die Ergebnisse der hochauflösenden Massenspektrometrie (FAB-MS, Quadrupol-Electrospray-MS, CID-MS/MS) sowie durch hochauflösende 600 MHz-Protonen- und 150 MHz-¹³C-NMR Korrelations-Spektroskopie, bewiesen.

Die antibakteriellen Wirkungen von HKI10311129 können durch die dem Fachmann bekannten Methoden, wie beispielsweise Agarplattendiffusionstest und die Bestimmung der minimalen Hemmkonzentration (MHK) bestimmt werden.

HKI10311129 ist gegen Bakterien, vorzugsweise gegen grampositive Bakterien wie z.B. *Bacillus subtilis* ATCC 6633, *Staphylococcus aureus* SG 511, *Staphylococcus aureus* 134/93 (multiresistent) und *Enterococcus faecalis* 1528 (vanA determinierte Vancomycin-Resistenz) wirksam.

Das erfindungsgemäße Antibiotikum HKI10311129 eignet sich aufgrund seiner sehr wertvollen, antibakteriellen Eigenschaften sehr gut zur Anwendung als antimikrobiell wirksames Therapeutikum, insbesondere in Anwendung gegen vanA-resistente Risikokeime, wie vanA-resistente Enterokokken und mittlerweile aufgetretene, multiresistente und vanA-determinierte Vancomycin-resistente Staphylokokken.

Das erfindungsgemäße Antibiotikum HKI10311129 kann als solches in Substanz oder als pharmazeutische Zubereitung mit geeigneten, dem Fachmann bekannten Hilfsstoffen oder Trägermaterial verabreicht werden. Die Zubereitungsformen werden in an sich bekannter Weise unter Verwendung üblicher Hilfs- und Trägerstoffe hergestellt, wie beispielsweise im "Remington's Pharmaceutical Sciences Handbook, Hack Pub. co., N. Y., USA" beschrieben. Bei den pharmazeutischen Zubereitungsformen kann es sich um Peroralia, z.B. Tabletten, Kapseln und Dragees, um perkutane Zubereitungsformen, z.B. Salben oder Sprays, Transdermale Therapeutische Systeme (TTS) oder Gele, um intranasale Zubereitungsformen wie Nasenspray oder Nasentropfen, rektale Zubereitungsformen wie Suppositorien und um Parenteralia, z.B.: Implantate, Presslinge und Ampullen handeln. Die erfindungsgemäßen Arzneimittel werden im allgemeinen oral oder parenteral appliziert, aber auch eine rektale Anwendung ist möglich. Geeignete feste oder flüssige galenische Zubereitungsformen sind beispielsweise Granulate, Pulver, Tabletten, Dragees, Suspensionen.

### Antimikrobielle Eigenschaften von HKI10311129:

Die antimikrobiellen Eigenschaften wurden nach den US Laborstandard - Methoden des Nationalen Komitee für Klinische Laborstandards (NCCLS) für aerob wachsende Bakterien in Flüssigmedien (National Comittee for Clinical Laboratory Standards, Methods for dilution antimicrobial susceptibility tests for bacteria that grow aerobically, Approved Standard M7-A. NCCLS, Villanova, Pa., 1991) geprüft. Die ermittelten MHK-Werte zeigt Tabelle 3.

**Tabelle 1: Physilcochemische Eigenschaften von HKI10311129**

| | |
|---|---|
| | |
| Eigenschaften | Gelb-orange amorphe Substanz |
| Molekulargewicht | 1113 |
| HRESI-MS ([M+Na]⁺) | 1136,4707 (ber. 1136,4678 für C₅₆H₇₅NaNO₂₂) |
| Formel | C₅₆H₇₅NO₂₂ |
| IR-Spektrum λₘₐₓ (cm⁻¹) | 858, 916, 997, 1053, 1112, 1163, 1225, 1247, 1351, 1377, 1448, 1592, 1625, 1686, 2853, 2922, 3417 |
| [α]_{D} | + 15,1 ° |
| UV-VIS-Spektrum λₘₐₓ in nm (ε in M⁻¹cm⁻¹) | 242 (15013,3); 434 (1975,7) |
| Schmelzpunkt: | 155-159°C |

**Tabelle 2: ¹³C und ¹H Signale im NMR-Spektrum von HKII0311129 (in CDCl₃; chemische Verschiebung in ppm; Multiplizität: m: Multiplett, s: Singulett; d: Dublett; q: quartemer; br: Breit*: Protonensignale aufgrund Überlagerung nicht zuzuordnen)**

| δ_{C} | δ_{H} | δ_{C} | δ_{H} | δ_{C} | δ_{H} |
|---|---|---|---|---|---|
| 17,09 | 1,15d | 56,71 | 3,90s | 102,23 | 4,63dd |
| 17,14 | 1,09d | 66,75 | 4,04m | 102,95 | 4,42dd |
| 17,19 | 1,09d | 66,75 | 3,86m | 110,46 | 6,11s |
| 17,98 | 0,70 d | 66,94 | 3,93m | 112,74 | q |
| 18,10 | 1,25 d | 67,50 | 3,56brs | 120,36 | 7,52s |
| 18,26 | 1,20 d | 71,34 | 3,23m | 124,85 | q |
| 22,66 | 1,28m/* | 72,18 | 4,32d | 133,53 | q |
| 23,60 | 1,88 m/1,65 m | 74,40 | 4,04m | 152,81 | q |
| 24,44 | * | 74,56 | 3,00m | 160,29 | q |
| 24,65 | 1,88m/1,60m | 74,93 | 3,31m | 163,16 | q |
| 24,65 | 2,05m/1,70m | 75,13 | 3,08m | 173,63 | q |
| 24,79 | 1,40m/* | 75,79 | 3,20m | 179,11 | q |
| 27,81 | 3,83m/3,50m | 78,75 | 3,07m | 189,29 | q |
| 29,68 | 1,34m/1,24m | 79,60 | 3,08m | 189,60 | q |
| 30,04 | 2,17m/* | 85,36 | q | 190,36 | q |
| 30,20 | * | 99,16 | 4,78s | 193,92 | q |
| 30,86 | 2,00m/1,55m | 99,39 | 4,80s | | |
| 30,98 | 1,81m/1,55m | 99,58 | 4,80s | | |
| 31,20 | 1,41m/* | 99,75 | 4,55dd | | |
| 43,02 | 3,08m | 101,04 | q | | |

**Tabelle 3: Antimikrobielle Aktivität des Antibiotikums HKI10311129**

| Testorganismus | MHK - Werte (µg/ml) |
|---|---|
| *Staphylococcus aureus* SG 511 | 0,8 |
| *Staphylococcus aureus* 134/93 (MRSA) | 0,8 |
| *Enterococcus faecalis* 1528 (VRE) | 0,4 |
| *Bacillus subtilis* ATCC 6633 | <0,05 |

### 1. Fermentation von Streptomyces sp. DSM 13059

### 1a. Züchtungsbedingungen für Streptomyces sp. DSM 13059:

Zur Gewinnung eines gut versporten Myzels wird Streptomyces sp. DSM 13059 6 Tage bei 28°C auf Nährboden folgender Zusammensetzung kultiviert (g/l):Hafermehl 20, Agar 20, pH 6,8-7,0 (Sterilisation 25 Min. bei 110°C).

### 1b. Herstellung einer Vorkultur des Stammes DSM 13059:

1 AgarMyzelstück (1-2cm² Oberfläche) der Ausgangskultur, die entsprechend 1a angezüchtet wurde, dient zur Beimpfung von 100ml eines Vorkulturmediums folgender Zusammensetzung (g/l): Sojabohnenmehl 15, Glucose 15, NaCl 5, CaCO₃ 1, KH₂PO₄ 3, (pH 6,5 nach Sterilisation 25 Min.bei 110°C) in 500ml Erlenmeyerkolben. Die beimpften Vorkulturen werden 48 Std. bei 28° C und 180 U/min. kultiviert.

### 1c. Herstellung einer Produktionskultur des Stammes DSM 13059:

5 ml dieser Vorkultur dienen zur Beimpfung von 500 ml Erlenmeyerkolben mit 100 ml eines Produktionsmediums folgender Zusammensetzung (g/1): Mannit 20, Sojabohnenmehl 20, pH 6,5 (nach Sterilisation 25 Min. bei 115°C). Die Kultivierung erfolgt 120 Stunden bei 28° C und 180 U/min auf einem Rundschwingtisch.

### 2. Gewinnung des Antibiotikums HKI 10311129 aus der Kulturbrühe

Die Kulturlösung gemäß Beispiel 1 wird durch Separieren in Myzel und Kulturfiltrat getrennt. Ersteres wird mit Methanol extrahiert. Der Extrakt wird mit dem 4-fachen Volumen an Wasser verdünnt und mit Ethylacetat extrahiert. Das Kulturfiltrat wird ebenfalls im Verhältnis 2:1 (wässrige Phase zu Ethylacetat) extrahiert. Die Ethylacetatextralcte werden vereinigt, über Natriumsulfat getrocknet und im Vakuum zur Trockne eingeengt. Chromatographische Aufreinigung von HKI 10311129: Der verbliebene braune, ölige Rückstand wird in wenig Chloroform aufgenommen und die Lösung wird filtriert. Durch Zugabe von Hexan (20-faches Volumen) wird rohes Produkt ausgefällt. Die weitere Aufreinigung erfolgt durch Gelpermeationscbromatographie an Sephadex LH-20 unter Verwendung von Methanol als Eluent.

Die antibakterielle Wirksamkeit aufweisende mittlere Fraktion wird vereinigt und im Vakuum zur Trockne eingeengt.

Die Feinreinigung erfolgt durch Kieselgelchromatographie unter Verwendung eines Chloroform-Methanol-Gradienten (100:0; 95:5; 9:1). Die gelbe Fraktion mit antibakterieller Wirkung wird zur Trockne eingeengt. Hochreines HKI 10311129 kann durch Chromatographie an Reversphasenkieselgel unter Verwendung eines schwach sauren Eluenten (z.B. MeCN-H₂O; 83:17 und 0,05 % Trifluoressigsäure) erhalten werden.

## Patentansprüche

1. Verbindung (HKI10311129) mit der Molmasse 1113 und der Summenformel C₅₆H₇₅NO₂₂, wobei folgende Strukturformel gilt:

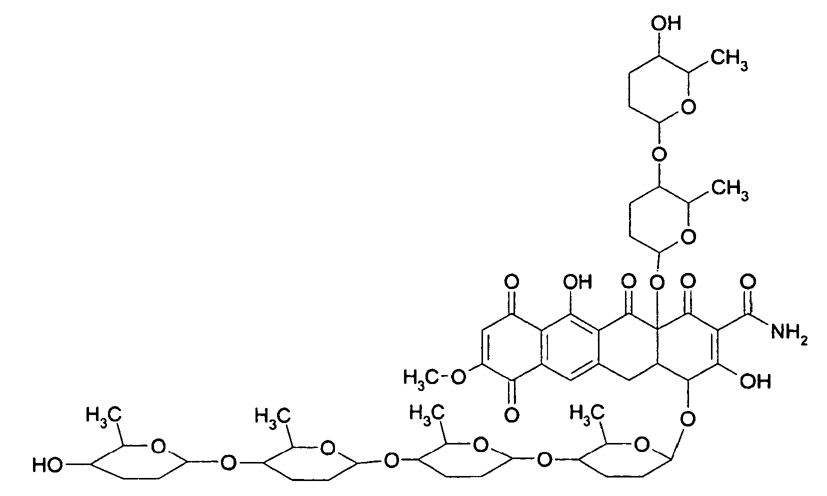

2. Arzneimittel enthaltend HKI10311129 gemäß Anspruch 1 nebst üblichen Träger- und Hilfsstoffen.

3. Verfahren zur Herstellung von HKI10311129, das **dadurch gekennzeichnet ist, dass** man *Streptomyces* sp. DSM 13059 (HKI 0179) in einem Nährmedium kultiviert, bis sich HKI10311129 in der Kulturbrühe anhäuft, und man es nachfolgend daraus isoliert.

4. Verwendung von HKI10311129 gemäß Anspruch 1 zur Herstellung von Arzneimitteln oder Zubereitungen zur Behandlung von bakteriellen Infektionskrankheiten insbesondere solche verursacht durch grampositive Erreger mit Resistenzen gegen gebräuchliche Antibiotika.

## Claims

1. Compound (HKI10311129) with the molar mass 1113 and the total formula C₅₆H₇₅NO₂₂, with the following structural formula applying:

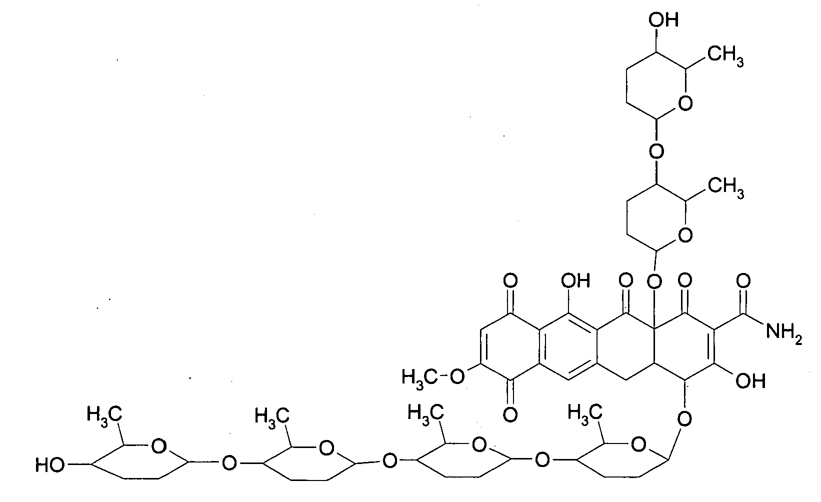

2. Medicament containing HKI10311129 according to claim 1 plus common carrier materials and additives.

3. A procedure for producing HKI10311129, wherein streptomyces sp. DSM 13059 is cultivated in a growth medium until HKI10311129 accumulates in the culture solution and is afterwards isolated from it.

4. Use of HKI10311129 according to claim 1 for producing medicaments or preparations for treating bacterial infectious diseases, in particular diseases that have been caused by gram-positive pathogens resistant against commonly available antibiotics.

## Revendications

1. Composé (HKI10311129) présentant la masse moléculaire 1113 et la formule chimique totale C₅₆H₇₅NO₂₂, dont la formule structurale est

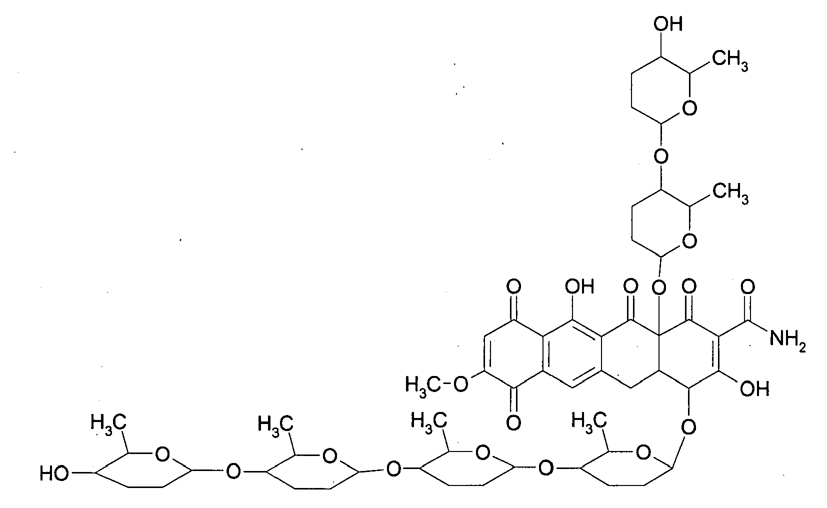

2. Médicament contenant HKI10311129 selon la revendication 1 ainsi que des supports et adjuvants habituels.

3. Procédé destiné à la préparation de HKI10311129, **caractérisé en ce que** *Streptomyces* sp. DSM 13059 (HKI0179) sont cultivées dans un milieu nutritif jusqu'à ce que HKI10311129 s'accumule dans un bouillon de culture pour être isolé par la suite.

4. Utilisation de HKI10311129 suivant la revendication 1 pour l'élaboration de médicaments ou de préparations servant au traitement de maladies infectieuses d'origine bactérienne, en particulier dans le cas où elles sont causées par des agents grampositifs présentant des résistances aux antibiotiques habituels.
